# EUROPEAN PATENT SPECIFICATION

(11) **EP 4 135 751 B1**
(45) Date of publication and mention of the grant of the patent: **31.07.2024**
(21) Application number: 21728830.7
(22) Date of filing: 13.04.2021
(51) Int. Cl.: A61K 38/20, A61K 47/18, A61K 47/26, C07K 14/54, C07K 14/78, C07K 16/18, A61K 9/00, A61K 9/08, A61K 47/68

(54) **FORMULATIONS OF COMPOSITIONS COMPRISING A RECOMBINANT PROTEIN COMPRISING INTERLEUKIN-12 AND AN ANTIBODY BINDING THE EXTRA-DOMAIN B OF FIBRONECTIN**
FORMULIERUNGEN VON ZUSAMMENSETZUNGEN MIT EINEM REKOMBINANTEN PROTEIN MIT INTERLEUKIN-12 UND EINEM ANTIKÖRPER ZUR BINDUNG DER EXTRAZELLULÄREN DOMÄNE B VON FIBRONECTIN
FORMULATIONS DE COMPOSITIONS COMPRENANT UNE PROTÉINE RECOMBINANTE COMPRENANT DE L'INTERLEUKINE-12 ET UN ANTICORPS SE LIANT AU DOMAINE EXTRACELLULAIRE B DE LA FIBRONECTINE

(30) Priority: 14.04.2020 EP 20169469
(43) Date of publication of application: 22.02.2023
(73) Proprietor: Philogen S.p.A., 53100 Siena (IT)
(72) Inventor: BACCI, Camilla, 53035 Monteriggioni (SI) (IT); HEMMERLE, Theresa, 8112 Otelfingen (CH)
(74) Representative: Michalski Hüttermann & Partner Patentanwälte mbB
(86) International application number: PCT/EP2021/059570
(87) International publication number: WO 2021/209452

(56) References cited:
- WO-A1-2018/011404
- WO-A1-2019/154986
- WO-A2-2006/119897
- S. M. RUDMAN ET AL: "A Phase 1 Study of AS1409, a Novel Antibody-Cytokine Fusion Protein, in Patients with Malignant Melanoma or Renal Cell Carcinoma", CLINICAL CANCER RESEARCH, vol. 17, no. 7, 29 March 2011 (2011-03-29), US, pages 1998 - 2005, XP055594696, ISSN: 1078-0432, DOI: 10.1158/1078-0432.CCR-10-2490
- M. MATASCI: "Abstract 5553: A novel immunocytokine for the treatment of cancer", CANCER RESEARCH, 1 July 2018 (2018-07-01), XP055720611, Retrieved from the Internet <URL:https://cancerres.aacrjournals.org/content/78/13_Supplement/5553> [retrieved on 20200806]

## Description

### Field of the invention

The present invention relates to a pharmaceutical formulation comprising a recombinant protein comprising (i) interleukin-12 (IL-12) and (ii) an antibody binding the extra-domain B (ED-B) of fibronectin, or a target binding fragment thereof, the formulation comprising:
- Histidine, Sucrose and EDTA, adjusted to have a pH of 8.0 ± 0.3;
- citric acid, sodium citrate, Sucrose, Glycerol, EDTA, adjusted to have a pH of 6.0 ± 0.3; or
- Hepes, NaCl, Mannitol, Glycerol, EDTA, adjusted to have a pH of 7.0 ± 0.3.

### Background

IL-12 is a heterodimeric cytokine comprising two disulfide-linked subunits, p35 and p40. IL-12 stimulates the production of IFNγ from T-cells and natural killer cells, and also induces differentiation of Th1 helper cells. IL-12 is a key mediator of innate and cell-mediated immunity, with the potential for anti-cancer and anti-metastatic activity.

Like many other cytokines, however, the administration of IL-12 is associated with severe toxicity (Car et al., 1999), even at doses as low as 1µg per kg per day, discouraging its development as an anticancer drug.

A number of cytokines have shown beneficial effects in preclinical animal models of cancer and immune disorders and represent promising agents for therapy. However, despite encouraging results, only few cytokines are approved as drugs (e.g., interleukin 2 (IL2, Proleukin^{®}), tumor necrosis factor (TNF, Beromun^{®}), interferon alpha (IFNα, Roferon A^{®} and Intron A^{®})). (Gutbrodt and Neri, 2012). Current indications in cancer include metastatic renal cell cancer, malignant melanoma, hairy cell leukemia, chronic myeloid lymphoma, sarcoma and multiple myeloma, either as single agents or in combination with chemotherapy. In addition, certain cytokines are used for the treatment of viral and bacterial infections in the clinic and are administered to patients suffering from chronic inflammatory conditions. Unfortunately, considerable toxicities can be observed at low doses, which prevent escalation to therapeutically active regimens.

Finding the right dose or dosage regimen for an immunocytokine which is for human therapy is complicated. The skilled artisan cannot rely on experience made with other biopharmaceutical drugs, like e.g. antibodies, due to the completely different modes of action, and the added toxicity issue. Hence, it is one object of the present invention to find a pharmaceutical formulation comprising a recombinant protein comprising (i) interleukin-12 (IL-12) and (ii) an antibody binding the extra-domain B (ED-B) of fibronectin.

The formulation of recombinant proteins for therapeutic use, like, e.g., antibodies, is a complex optimization process utilizing unique pharmaceutical additives to address the varying demands of storage and route of administration necessary for the clinical application.

WO 2019/154986 A1 describes the production of two diabody-II, 12 conjugates IL12-L19L19 (with 2 different linkers, same as in the present application) and their use in the treatment of patients with melanoma, preferably patients demonstrating disease progression on an immune checkpoint blocking therapy based regimen.

WO 2018/011404 A1 is directed to compositions for treating cancer, preferably melanoma or carcinoma, using cytokines conjugated to an antibody targeting fibronectin (L19). The preferred embodiments are L19-IL2 and L19-TNFa. Stability tests are carried out with using several different buffer and pH formulations. WO2018/011404 does not disclose conjugates comprising IL-12.

Immunocytokines are recombinant proteins comprising a protein-based binding molecule, mostly an antibody, and a cytokine. On that basis, immunocytokines vary significantly, in their chemico-physical properties, from antibodies. This applies to, for example, the domain structure, the molecular weight, or the number of inter- and intrachain disulfide bridges, and so on. As a consequence, the solubility, the aggregation behavior, and the pharmacodynamics of immunocytokines may vary significantly from those known from antibodies. Lessons learned from antibodies can hence not simply be transferred to immunocytokines.

All this makes clear that finding the formulation for an immunocytokine which is for human administration is not straightforward. Hence, it is one object of the present invention to find a formulation for an immunocytokine comprising (i) interleukin-12 (IL-12) and (ii) an antibody binding the extra-domain B (ED-B) of fibronectin.

### Brief description of the drawings and tables

The term IL12-L19L19 designates the recombinant protein comprising (i) interleukin-12 (IL-12) and (ii) an antibody binding the extra-domain B (ED-B) of fibronectin, or a target binding fragment thereof, which is provided in the single chain diabody format (also called "tandem diabody") as explained herein. The recombinant protein comprises the amino acid according to SEQ ID NO: 9.
Table 1. Dosage Regimen.
Table 2. Total dosages for patients with different body weights (kg) per each administration in µg, based on the different dosage units/dosage regimen (µg/kg⁻¹).
Table 3. Formulations.
Table 4. Sequences.
Table 5. IL12-L19L19 Formulation Study. Storage temperatures, time-points, assays and acceptance criteria are detailed.
Table 6. IL12-L19L19 Formulation Study. Summary of the steps yield (formulation and concentration steps) and of the single chain diabody purity, evaluated by SEC both after formulation and after concentration up to 2mg/ml.
Table 7. IL12-L19L19 Formulation Study. A₂₈₀ₙₘ and SEC results collected for each sample and for each time-point, both after stressing conditions (i.e. 3 repeated cycles of freezing/thawing) and at 2-8°C storage temperature.
Table 8. IL12-L19L19 Formulation Study. Stability study under stress conditions: evaluation of the loss in A₂₈₀ₙₘ after the 3^{rd} cycle of freezing/thawing and classification of the samples from the best to the worst, based on this loss.
Table 9. IL12-L19L19 Formulation Study. Stability study at 2-8°C: evaluation of the loss in A₂₈₀ₙₘ after 14 days of storage and classification of the samples from the best to the worst, based on this loss.

Figure 1. IL12-L19L19 Formulation Study. SEC analysis of the different formulated samples, before and after the concentration step.
Figure 2. IL12-L19L19 Formulation Study. SEC analysis of the different formulated samples, before and after the concentration step.

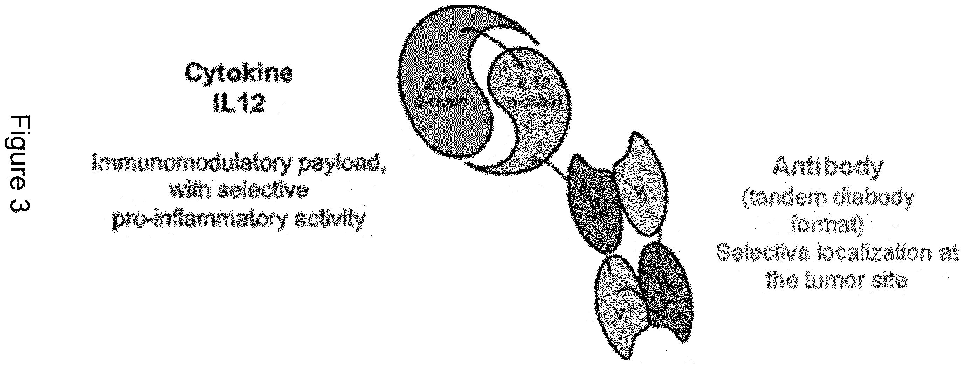

Figure 3. Schematic drawing of IL12-L19L19. The molecule consists of a single-chain polypeptide consisting of the two subunits of the immunomodulatory payload IL12 fused to a human vascular targeting antibody in a single-chain diabody.

### Summary of the Invention

The present invention provides formulations of compositions comprising a recombinant protein comprising (i) interleukin-12 (IL-12) and (ii) an antibody binding the extra-domain B (ED-B) of fibronectin, or a target binding fragment or derivative thereof.

The invention and general advantages of its features will be discussed in detail below. The invention is defined by the features of the independent claim.

According to one aspect, the present invention relates to a pharmaceutical formulation comprising a recombinant protein comprising (i) interleukin-12 (IL-12) and (ii) an antibody binding the extra-domain B (ED-B) of fibronectin, or a target binding fragment thereof, the formulation comprising:
- Histidine, Sucrose and EDTA, adjusted to have a pH of 8.0 ± 0.3;
- citric acid, sodium citrate, Sucrose, Glycerol, EDTA, adjusted to have a pH of 6.0 ± 0.3; or
- Hepes, NaCl, Mannitol, Glycerol, EDTA, adjusted to have a pH of 7.0 ± 0.3.

Disclosed herein, but not part of the invention, is a dosage unit is provided, comprising ≥ 0.1 µg kg⁻¹, relative to a human patient's body weight, of a recombinant protein comprising (i) interleukin-12 (IL-12) and (ii) an antibody binding the extra-domain B (ED-B) of fibronectin, or a target binding fragment or derivative thereof.

In Rudman *et al.* 2011, AS1409 is disclosed, which is a fusion protein comprising the humanised antibody BC1 in IgG format linked to interleukin-12 (IL-12). It is designed to deliver IL-12 to tumor-associated vasculature using an antibody targeting the ED-B variant of fibronectin. A phase 1 trial of weekly infusional AS1409 was carried out in renal carcinoma and malignant melanoma patients. Safety, efficacy, markers of IL-12-mediated immune response, and pharmacokinetics were evaluated. Doses of 15µg/kg and 25µg/kg were studied. The study demonstrated the safety of this approach, and provided pharmacodynamic support for the proposed mechanism of action. Along with evidence of efficacy against metastatic melanoma, it was stipulated that the experiments provided a rationale for progression to a phase II trial. However, no phase II trial was ever launched.

Generally, because immunocytokines have a potentiating effect on the immune system - with each cytokine being capable of stimulating two or more immune cells - finding of a safe and efficient dosage is much more difficult than *e.g.* in antibody therapy, where the abundance of a given target - either a ligand that is to be inactivated, or a receptor that is to be blocked - is known, and a clear stochiometric relationship between antibody and target can be calculated.

Strauss *et al.* (2018) disclose a First-In-Human Phase I Trial of a Tumor-Targeted Cytokine (NHS-IL12) in subjects with Metastatic Solid Tumors. The NHS-IL12 immunocytokine is composed of two IL-12 heterodimers, each fused to one of the H chains of the anti-histone antibody NHS76. The maximum tolerated dose (MTD) was determined to be 16.8 µg/kg. Because NHS76 has, via the histones, affinity for both single- and double-stranded DNA, NHS-IL12 targets delivery to regions of tumor necrosis where DNA has become exposed.

The teachings from this study cannot be transferred to the present IL12-anti EDB recombinant protein, because of at least the fact that histones, as targets for the NHS antibody, are intracellular targets, compared to EDB, which is an extracellular target, and because histones have a different abundance in tumor tissue than EDB.

In Puca *et al.* (2019), the cloning and characterization of a novel fusion protein (termed L19-mIL12) is disclosed. The fusion protein consists of murine interleukin-12 in single-chain format, sequentially fused to the anti EDB antibody L19 in a single-chain diabody (also defined as "tandem diabody") format. The authors reported that in mice, L19-mIL12 was very well tolerated at a dose of 12 µg, which is equivalent to the human dose of 2 mg, under consideration of the body surface scaling factor - a dose which is by far higher that what has been reported by Rudman *et al.* and Straus *et al.*

Disclosed herein, but not part of the invention, is a dosage unit or regimen, comprising ≤ 100 µg kg⁻¹, relative to a human patient's body weight, of a recombinant protein comprising (i) interleukin-12 (IL-12) and (ii) an antibody binding the extra-domain B (ED-B) of fibronectin, or a target binding fragment or derivative thereof.

Disclosed herein, but not part of the invention, is a recombinant protein, comprising (i) interleukin-12 (IL-12) and (ii) an antibody binding the extra-domain B (ED-B) of fibronectin, or a target binding fragment or derivative thereof, for use in the treatment of a patient being diagnosed for, or suffering from, cancer, wherein the recombinant protein is administered in a dose according to the above description.

The following dosage regimen of a recombinant protein comprising (i) interleukin-12 (IL-12) and (ii) an antibody binding the extra-domain B (ED-B) of fibronectin, or a target binding fragment or derivative thereof, relative to a human patient's body weight, provide a good compromise between good efficacy and reduced side effects.

**Table 1:**

| **µg kg⁻¹** | **half weekly** | **weekly** | **biweekly** | **triweekly** | **monthly** |
|---|---|---|---|---|---|
| 0,5 | dosage regimen can be administered 1x, 2x, 3x, 4x, 5x, 6x, 7x or 8x | | | | |
| 0,75 | dosage regimen can be administered 1x, 2x, 3x, 4x, 5x, 6x, 7x or 8x | | | | |
| 1 | dosage regimen can be administered 1x, 2x, 3x, 4x, 5x, 6x, 7x or 8x | | | | |
| 1,25 | dosage regimen can be administered 1x, 2x, 3x, 4x, 5x, 6x, 7x or 8x | | | | |
| 1,5 | dosage regimen can be administered 1x, 2x, 3x, 4x, 5x, 6x, 7x or 8x | | | | |
| 1,75 | dosage regimen can be administered 1x, 2x, 3x, 4x, 5x, 6x, 7x or 8x | | | | |
| 2 | dosage regimen can be administered 1x, 2x, 3x, 4x, 5x, 6x, 7x or 8x | | | | |
| 2,25 | dosage regimen can be administered 1x, 2x, 3x, 4x, 5x, 6x, 7x or 8x | | | | |
| 2,5 | dosage regimen can be administered 1x, 2x, 3x, 4x, 5x, 6x, 7x or 8x | | | | |
| 2,75 | dosage regimen can be administered 1x, 2x, 3x, 4x, 5x, 6x, 7x or 8x | | | | |
| 3 | dosage regimen can be administered 1x, 2x, 3x, 4x, 5x, 6x, 7x or 8x | | | | |
| 3,25 | dosage regimen can be administered 1x, 2x, 3x, 4x, 5x, 6x, 7x or 8x | | | | |
| 3,5 | dosage regimen can be administered 1x, 2x, 3x, 4x, 5x, 6x, 7x or 8x | | | | |
| 3,75 | dosage regimen can be administered 1x, 2x, 3x, 4x, 5x, 6x, 7x or 8x | | | | |

As shown in the table, any one of the above dosage regimens can be administered 1x, 2x, 3x, 4x, 5x, 6x, 7x or 8x, based on the suggested interval of half weekly to monthly.

Hence, disclosed herein, but not part of the invention, is a dosage unit or regimen comprising between ≥ 0,5 µg kg⁻¹ and < 4 µg kg⁻¹, relative to a human patient's body weight, of a recombinant protein comprising (i) interleukin-12 (IL-12) and (ii) an antibody binding the extra-domain B (ED-B) of fibronectin, or a target binding fragment or derivative thereof, is provided.

The following Table 2 shows total dosages for patients with different body weights (kg) per each administration in µg, based on the different dosage units/dosage regimen (µg/kg⁻¹).

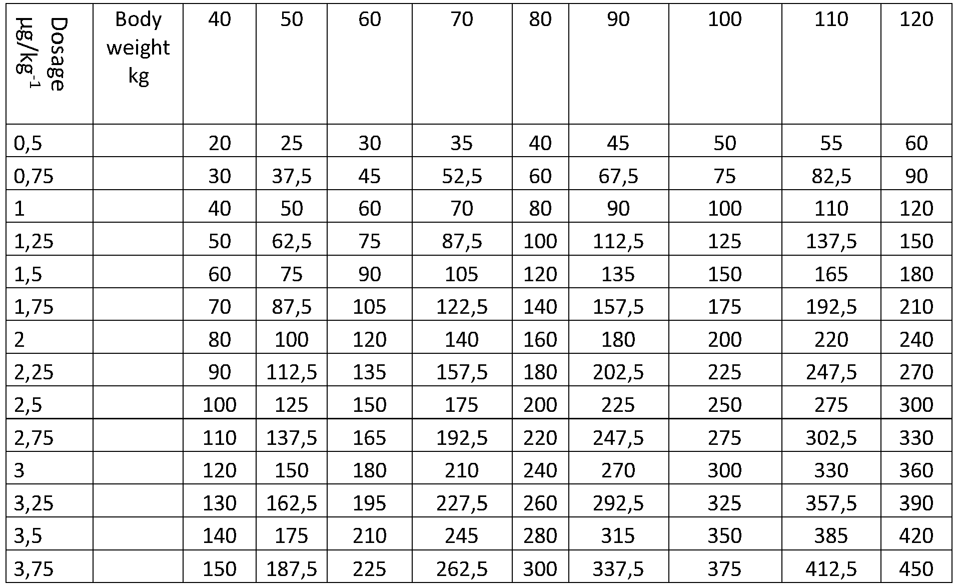

Disclosed herein, but not part of the invention, is a recombinant protein, comprising (i) interleukin-12 (IL-12) and (ii) an antibody binding the extra-domain B (ED-B) of fibronectin, or a target binding fragment or derivative thereof, for (use in) the treatment of a patient being diagnosed for, or suffering from, cancer. Disclosed herein, but not part of the invention, is that the cancer is advanced/metastatic immunotherapy responsive solid carcinoma or lymphoma.

As part of the present disclosure, the above conditions can as well be combined.

Disclosed herein, but not part of the invention, is the recombinant protein for use, wherein the carcinoma or lymphoma has progressed on immune checkpoint-blockade therapy,

Disclosed herein, but not part of the invention, is the recombinant protein for use, wherein the carcinoma or lymphoma has an Eastern cooperative oncology group (ECOG) performance status ≤ 2.

Disclosed herein, but not part of the invention, is the recombinant protein for use, wherein the carcinoma or lymphoma is characterized by at least one unidimensionally measurable lesion either by computed tomography (CT), MRI or PET/CT as defined by RECIST (v. 1.1) for solid tumors or by LUGANO criteria for malignant lymphoma.

As part of the present disclosure, the above conditions can as well be combined.

Disclosed herein, but not part of the invention, is the recombinant protein for use,wherein the patient has received an immune checkpoint blockade therapy-based regimen as immediate prior treatment.

Disclosed herein, but not part of the invention, is the recombinant protein for use, wherein the patient has had clinical benefit (CR/PR/SD) while on immune checkpoint blockade therapy defined as ≥ 3 month free from progression from initial imaging documenting metastatic disease followed by radiographic disease progression after immune checkpoint blockade therapy.

Disclosed herein, but not part of the invention, is the recombinant protein for use, wherein the patient has received ≥2 prior systemic therapies, when being diagnosed for, or suffering from, DLCBL.

Disclosed herein, but not part of the invention, is the recombinant protein for use, wherein the patient has been negatively tested for HIV, HBV and HCV.

The terms CR/PR/SD, as used herein, relate to responses as defined by the Response Evaluation Criteria In Solid Tumors (RECIST) criteria. Complete response (CR) = Disappearance of all target lesions, Partial response (PR) = at least a 30% decrease in the sum of the LD of target lesions, taking as reference the baseline sum LD, and Stable disease (SD) = Neither sufficient shrinkage to qualify for PR nor sufficient increase to qualify for PD, taking as reference the smallest sum LD since the treatment started.

The term "ECOG performance status", as used herein, is a score which describes a patient's level of functioning in terms of their ability to care for themselves, daily activity, and physical ability (walking, working, etc.), as developed by the Eastern Cooperative Oncology Group.

The term "immune checkpoint-blockade therapy" as used herein, relates to a therapy that uses medications known as immune checkpoint inhibitors to address several types of cancer. Specifically, these medications can help the body's immune system recognize and attack cancerous cells. Immune checkpoint inhibitors include inhibitors or antagonists to inter alia CTLA-4, PD-1, PD-L1, LAG 3, TIM3 and OX40. Some well-established immune checkpoint inhibitors are Ipilimumab, Nivolumab, Pembrolizumab, Atezolizumab, Avelumab, Durvalumab and Cemiplimab.

The term "negatively tested for HIV, HBV and HCV" means that the patient has been tested negatively for infections with, or presence of antibodies against, HIV virus, Hepatitis B virus, or Hepatitis C virus.

According to one aspect of the invention, a pharmaceutical formulation is provided, comprising a recombinant protein comprising (i) interleukin-12 (IL-12) and (ii) an antibody binding the extra-domain B (ED-B) of fibronectin, or a target binding fragment thereof, the formulation comprising:
- Histidine, Sucrose and EDTA, adjusted to have a pH of 8.0 ± 0.3;
- citric acid, sodium citrate, Sucrose, Glycerol, EDTA, adjusted to have a pH of 6.0 ± 0.3; or
- Hepes, NaCl, Mannitol, Glycerol, EDTA, adjusted to have a pH of 7.0 ± 0.3.

In one embodiment, the formulation can comprise Histidine, Sucrose and EDTA, adjusted to have a pH of 8.0 ± 0.3. In another embodiment, the formulation can comprise citric acid, sodium citrate, Sucrose, Glycerol, EDTA, adjusted to have a pH of 6.0 ± 0.3. In one embodiment, the formulation can comprise Hepes, NaCl, Mannitol, Glycerol, EDTA, adjusted to have a pH of 7.0 ± 0.3.

These formulations satisfy the high demands as regards stability under stress conditions (low protein loss after several cycles of freezing/thawing and low aggregate formation at 2 - 8 °C).

AS1409 (Rudman *et al.,* discussed above), was supplied as a 1mg/ml solution in aqueous buffer at pH 6.0. It was administered to patients following a 1 to 1 dilution with 0.9% sodium chloride. For NHS-IL12 (Strauss *et al*., discussed above) no data regarding the formulation is given. L19-mIL12 (Puca *et al*., discussed above) was diluted in phosphate buffer saline.

While these formulations can arguably be used for scientific and clinical trials, they are certainly not suitable for the market, as no considerations have been devoted to issues like shelf life or aggregation. The following Table 3 shows six preferred formulations:

| **Formulation no** | **2B** | | **4A** | | **1B** | |
|---|---|---|---|---|---|---|
| alias | "Histidine buffer" | | "Citrate buffer" | | "Hepes buffer" | |
| Histidine mM | 5-40 | 20 | | | | |
| Hepes mM | | | | | 5-30 | 15 |
| Sucrose % w/v | 4-15 | 8,5 | 4-15 | 8 | | |
| Citric acid mM | | | 0.2-4 | 1 | | |
| Na citrate mM | | | 3-20 | 10 | | |
| EDTA | 20-100 mg/L | 50 mg/L | 2-10 mM | 5 mM | 2-10 mM | 5mM |
| Glycerol % w/v | | | 0.2-4 | 1 | 0.2-4 | 1 |
| Mannitol mM | | | | | 20-100 | 50 |
| NaCl mM | | | | | 10-80 | 30 |
| pH | 7-9 | 8.0 ± 0.3 | 5-6 | 6.0 ± 0.3 | 6-8 | 7.0 ± 0.3 |

Application WO2018011404A1 assigned to the applicant of the present invention discloses a formulation for an immunocytokine comprising the antibody L19 and the cytokine TNF. In example 4, disclosed therein on pages 25 - 27, the following formulations were inter alia investigated:
(i) Histidine buffers prepared at pH 6, 8 and 9:
   - Hist-1 comprises 20 mM histidine at pH 6.0, 8.5% Sucrose (w/v), 130 mM EDTA.
   - Hist-2 comprises 20 mM histidine at pH 8.0, 8.5% Sucrose (w/v), 130 mM EDTA.
   - Hist-3 comprises 20 mM histidine at pH 9.0, 8.5% Sucrose (w/v), 130 mM EDTA.
(ii) Citrate buffer prepared at pH 6 .6
   - Citrate-1 comprises 5.6 g/L sodium Citrate, 0.21 g/L citric acid, 70 g/L trehalose dihydrate, 0.2 g/L polysorbate80, 1% (w/v) glycerol, 5mM EDTA, pH 6.6.

In example 1, disclosed therein on pages 21 - 23, the following formulations were inter alia investigated:
(iii) Hepes buffers prepared at pH 7.5 and 8.0:
- Hepes-1 comprises 30 mM Hepes at pH 7.5, 5 mM EDTA, 75 mM mannitol and 1.8% glycerol (w/v)
- Hepes-2 comprises 30 mM Hepes at pH 7.5, 5 mM EDTA, 75 mM mannitol, 1.8% glycerol (w/v) and 0.1% polysorbate20
- Hepes-3 comprises 15 mM Hepes at pH 8.0, 5 mM EDTA, 75 mM mannitol and 1.8% glycerol (w/v)
- Hepes-4 comprises 15 mM Hepes at pH 8.0, 5 mM EDTA, 75 mM mannitol, 1.8% glycerol (w/v) and 0.005% polysorbate20
- Hepes-5 comprises 15 mM Hepes at pH 8.0, 5 mM EDTA, 75 mM mannitol, 1.8% glycerol (w/v) and 0.01% polysorbate20
- Hepes- 6 comprises 15 mM Hepes at pH 8.0, 5 mM EDTA, 75 mM mannitol, 1.8% glycerol (w/v) and 0.05% polysorbate20

For the Histidine buffers, no acceptance criteria were met. For the citrate buffer, both the visual clarity and A280 stability criteria were met but the purity criteria was not met indicating particles in suspension or aggregation of the trimer. Histidine and citrate buffers showed particles in suspension and were not considered for further investigation.

For Hepes formulations comprising < 0.1% polysorbate20, the acceptance criteria were not met either. Only for met. Hepes formulations comprising ≥ 0.1% polysorbate20, the acceptance criteria were met.

On that basis, it is highly surprising that the formulations according to the above table deliver acceptable results, even though they are highly similar to the formulations used in examples 1 and 4 of WO2018011404A1, and even though the recombinant protein of the present invention has structural similarity with L19-TNF of WO2018011404A1.

According to embodiments of the formulation of the invention, the antibody comprised in the recombinant protein comprises at least one single-chain Fv (scFv) antibody fragment, optionally a single chain diabody.

As used herein, the term "single chain diabody" relates to a construct of two single chain Fv (scFv) antibodies with a short linker, preferably 3 - 10 amino acids long, more preferably 5 amino acid long (also known as "diabodies"), joined to one another by a longer linker, preferably 5 - 20 amino acids long, more preferably 15 amino acid long, according to the following scheme (N->C orientation): L19VH-linker-L19VL-linker-L19VH-linker- L19VL.

According to embodiments of the formulation of the invention, the IL-12 comprised in the recombinant protein comprises a p40 subunit and a p35 subunit, linked by a linker.

According to embodiments of the formulation of the invention, the p40 subunit and the p35 subunit comprise the amino acid sequence according to SEQ ID NO: 1 or SEQ ID NO: 3, respectively.

According to embodiments of the formulation of the invention, the antibody comprised in the recombinant protein is the anti-EDB antibody L19, comprising the amino acid sequence according to SEQ ID NO: 5 as VL domain and SEQ ID NO: 7 as VH domain.

Disclosed herein, but not part of the invention, is the formulation, wherein at least one of the antibody, the IL-12 and/or the linker connecting the two is one disclosed in WO2019154986, optionally wherein the recombinant protein is one disclosed in WO2019154986.

WO2019154986 describes technical and physiological properties of the recombinant protein that is subject to the present invention.

According to embodiments of the formulation of the invention, the recombinant protein comprises
- a p40 domain linked to a p35 domain by a first linker;
- a first L19 VH domain linked to the p35 domain by a SAD linker;
- a first L19 VL domain linked to the first L19 VH domain by a third linker;
- a second L19 VH domain linked to the first L19 VL domain by a fourth linker;
- a second L19 VL domain linked to the second L19 VH domain by a fifth linker.

According to embodiments of the formulation of the invention, the recombinant protein comprises, optionally consists of, the amino acid sequence according to SEQ ID NO: 9.

According to embodiments of the formulation of the invention, administration is done intravenously or subcutaneously.

Disclosed herein, but not part of the invention, is the dosage unit or recombinant protein for use provided in a formulation according to the above description.

According to embodiments of invention, the formulation according to the above description, comprises a recombinant protein according to the above description.

Disclosed herein, but not part of the invention, is a kit of parts comprising
a) the dosage unit or recombinant protein for use according to the above description
b) an apparatus for administering the dosage unit or recombinant protein, and, optionally
c) instructions for use.

### Examples

In the claims, the word "comprising" does not exclude other elements or steps, and the indefinite article "a" or "an" does not exclude a plurality. Any reference signs in the claims should not be construed as limiting the scope.

All amino acid sequences disclosed herein are shown from N-terminus to C-terminus.

### Example 1: Toxicology Studies in Cynomolgous monkeys

The extradomain B of fibronectin, which is targeted by the L19 antibody in IL12-L19L19, is a very well conserved domain across species and is 100 % identical in man and monkeys.

The homology between human and monkey IL12 is 98% and 95% for p40 and p35, respectively, and human IL12 is active both in man and monkey. The activity of IL12-L19L19 has been tested on human and monkey peripheral blood mononuclear cells (PBMCs). IL12-L19L19 and recombinant human IL12 have comparable activities with regard to IFNγ increase on both human and monkey PBMCs. However, both IL12-L19L19 and recombinant IL12 are 10x less active in monkey than in human PBMCs.

In the toxicity study, monkeys have received eight weekly intravenous administrations of IL12-L19L19 at human equivalent doses (HED) of 13, 51 or 213 µg/kg/week. The tested doses were identified as safe for the respiratory, central nervous, cardiac and renal system since no IL12-L19L19 related observations were reported.

### Example 2: Formulation development

The formulation study was performed by screening several buffers, different in salts composition and pH. The following buffers were investigated:
1) Hepes buffer
   - 1A. 15mM Hepes, 50mM Mannitol, 1% w/v Glycerol, 5mM EDTA, pH 7.0
   - 1B. 15mM Hepes, 30mM NaCl, 50mM Mannitol, 1% w/v Glycerol, 5mM EDTA, pH 7.0
2) Histidine buffer
   - 2A. 20mM Histidine, 8,5% w/v Sucrose, 50mg/L EDTA, pH 6.0
   - 2B. 20mM Histidine, 8,5% w/v Sucrose, 50mg/L EDTA, pH 8.0
3) Phosphate buffer
   - 3A. 15mM NaH₂PO₄, 10mM Na₂HPO₄, 30mM NaCl, 50mM Mannitol, 1% w/v Glycerol, 5mM EDTA, pH 6.5
   - 3B.15mM NaH₂PO₄, 10mM Na₂HPO₄, 30mM NaCl, 50mM Mannitol, 1% w/v Glycerol, 5mM EDTA, pH 7.5
4) Citrate buffer
   - 4A. 1mM citric acid, 10mM sodium citrate, 8% w/v Sucrose, 1% w/v Glycerol, 5mM EDTA, pH 6.0

The study was performed by applying the following operative procedure:
1) The formulation is performed by desalting chromatography.
2) The formulated product is 0,22µm filtered before analysis
3) The concentration of the formulated protein is done by using Amicon device (10kDa cut-off) and centrifugation.
4) The product is formulated at 2mg/ml.

To accept the formulated product, before to start the stability study, a preliminary acceptance criterion related to the visual appearance was evaluated as follows:
- if the solution is cloudy and/or particles in suspension are visible → reject the sample.
- if the solution is clear and free of visible particles in suspension → perform the stability study.

The stability study was performed for each sample as summarized in Table 5, defining that:
- all time-points of the stability study must be analyzed even if the first data are out of specifications.
- A₂₈₀ₙₘ is evaluated after sample centrifugation.
- A₂₈₀ₙₘ pharmacopoeia method is applied (ref. Ph. Eur. curr. ed., paragraph 2.5.33).
- SEC analysis is performed using TSKgel G3000 SWXI, column

The material used to perform the formulation study was obtained by preparative SEC of IL12-L19L19: it was expressed by TGE procedure (i.e. Transient Gene expression) and purified on Protein A resin by eluting with TEA 100mM native pH, then it was desalted in PBS as described in WO2019154896.

Table 6 summarizes the recorded results.

The total yield (i.e., formulation + concentration steps) was between 86.2% and 90.2%, so very comparable data were recorded among the different formulation conditions investigated. The single chain diabody purity was between 95.49% and 96.11% after the formulation step. Considering that the purity of the input sample was 96.06%, the recorded data after the formulation attest that no aggregates formation was induced by the buffer exchange. After concentration up to 2 mg/ml, the SEC results show the % of single chain diabody between 93.15% and 94.89% showing a small increase of the high MW form (< 3%), due to the concentration procedure itself.

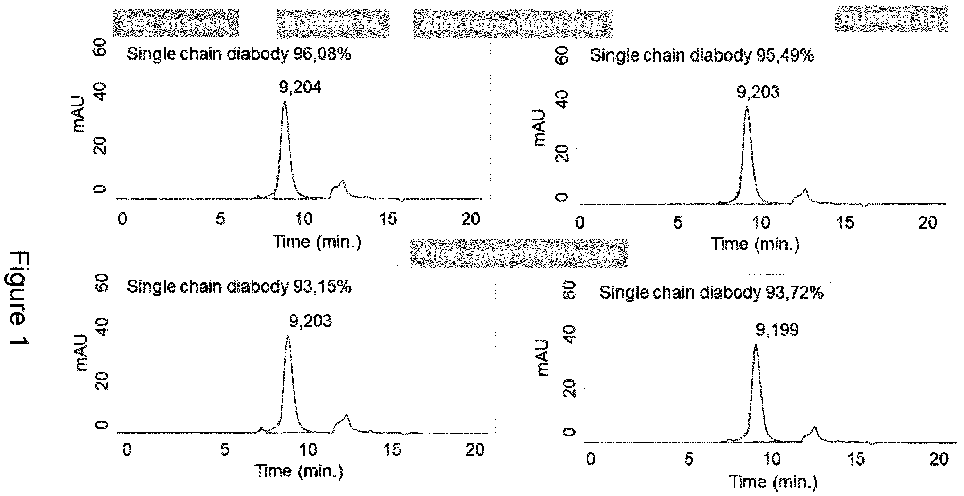

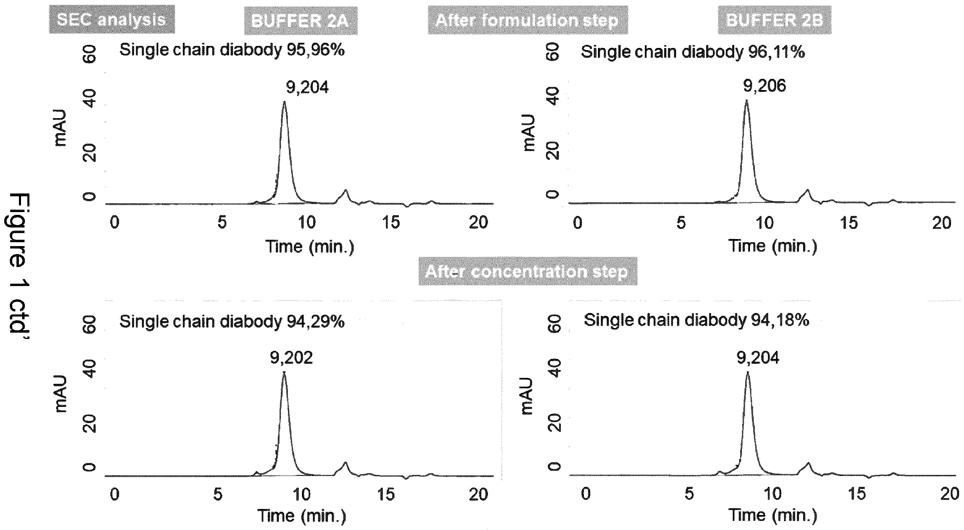

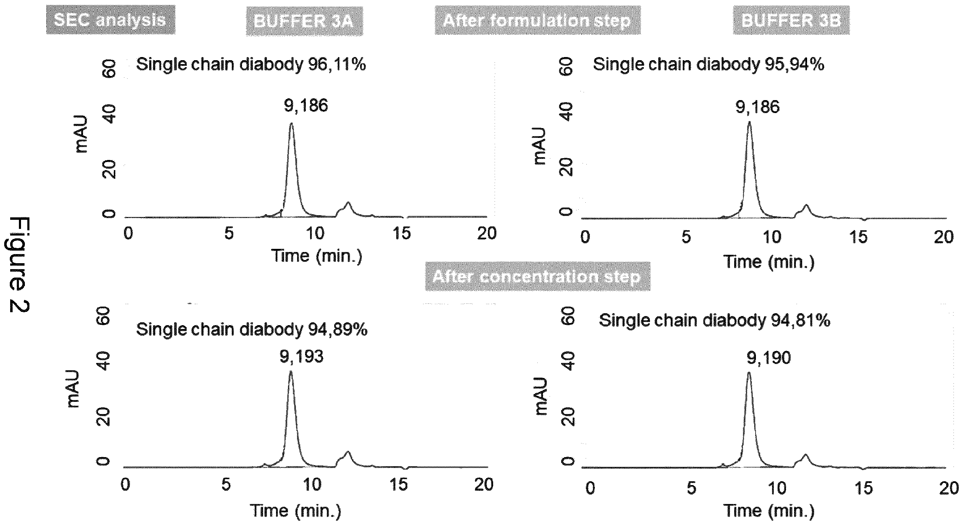

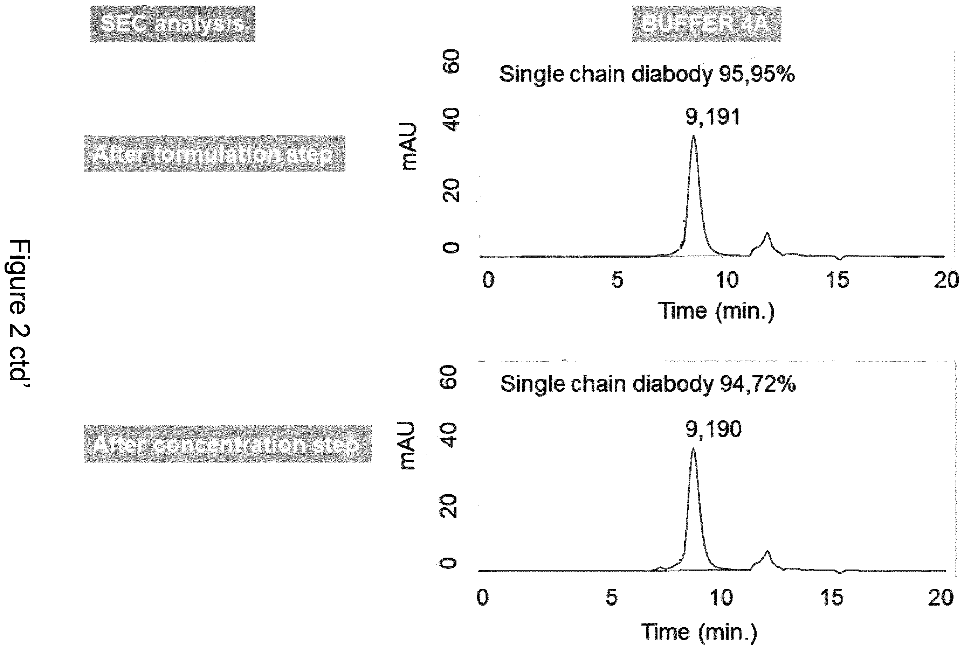

Figures 1 and 2 show the SEC profiles of the sample after formulation and after concentration.

The visual appearance of all formulated and concentrated samples was clear and free of visible particles, then the samples were investigated for their stability under stressing conditions (i.e. 3 repeated cycles of freezing at -80°C and thawing at RT) and at 2-8°C storage temperature.

Table 7 reports the recorded results during the stability study, for each formulated sample.

The stability of IL12-L19L19 was investigated in 7 formulation buffers under stress conditions (i.e. 3 cycles of freezing at -80°C and thawing at RT) and after 14 days of storage at 2-8°C. The visual appearance was very good for all samples.

The SEC profile and the % of the single chain diabody form were well comparable among all samples and only very small variations were observed in terms of decrease of diabody and increase of aggregates (reported as "HMW forms" in Table 7).

Based on the comparable results recorded for appearance and SEC, A₂₈₀ₙₘ values were used to compare the different formulations and to define the best conditions.

Table 8 reports the loss in A₂₈₀ₙₘ value after the 3rd cycles of freezing/thawing, recorded for each sample under study. The samples were classified from the best to the worst based on the loss in A₂₈₀ₙₘ.

The loss of protein, evaluated by A₂₈₀ₙₘ reading, seems to depend not only on the pH of the buffer, but, mostly, on the buffer excipients.

In details:
- Comparing the Histidine buffers: pH 8.0 (2B) is better than pH 6.0 (2A).
   In both buffers is 8.5% w/v Sucrose, in this case, the pH makes the difference.
- Comparing Histidine buffer pH 8.0 (2B) to Citrate buffer pH 6.0 (4A) the same stability was recorded.
   In both buffers is Sucrose (8.5% w/v and 8% w/v, respectively): sucrose seems to assure the stability and the pH is irrelevant.
- Comparing Hepes/NaCl buffer (1B) to Hepes buffer without NaCl (1A) → the presence of NaCl seems to help the stability.
- In phosphate buffer pH 6.5 (3A) the product stability is better than at pH 7.5 (3B), same composition buffer composition.

Based on the stress study, the following buffers were selected as good candidates:
2B. 20mM Histidine, 8.5% w/v Sucrose, 50mg/L EDTA, pH 8.0
4A. 1mM citric acid, 10mM sodium citrate, 8% w/v Sucrose, 1% w/v Glycerol, 5mM EDTA, pH 6.0
1B. 15mM Hepes, 30mM NaCl, 50mM Mannitol, 1% w/v Glycerol, 5mM EDTA, pH 7.0

Table 9 reports the loss in A₂₈₀ₙₘ value after 14 days of storage at 2-8°C, recorded for each sample under study. The samples were classified from the best to the worst based on the loss in A₂₈₀ₙₘ.

In detail:
- Histidine buffer pH 8.0 (2B) = Histidine buffer pH 6.0 (2A) = (very similar). Citrate buffer pH 6.0 (4A): also at 2-8°C, the presence of sucrose assures the stability, 2% protein loss was the maximum recorded in these cases.
   However, the buffers listed above showed the highest decrease of single chain diabody form: 2.4%.
- Hepes/NaCl buffer (1B) = Hepes buffer without NaCl (1A) showing 2.4-2.5% of protein loss.
- In phosphate buffer pH 7.5 (3B) the product stability was better than in the phosphate buffer pH 6.5 (3A) (same composition): 0.8% vs. 2.9% of protein loss respectively.

Based on the results recorded during the formulation study, the preferred formulation buffer was the buffer 2B: 20mM Histidine, 8.5% w/v Sucrose, 50mg/L EDTA, pH 8.0.

It showed the best results for the study under stress conditions (3.3% of protein loss after the 3rd cycle of freezing/thawing and no diabody loss in SEC analysis) and good results for the study at 2-8°C (only 2.1% of protein loss and 2.4% of loss of single chain diabody form with aggregates formation).

### References

Gutbrodt and Neri, Antibodies 2012, 1(1), 70-87
Rudman SM et al, Clin Cancer Res. 2011 April 1; 17(7): 1998-2005
Strauss J et al., Clin Cancer Res. 2019 Jan 1;25(1):99-109. doi: 10.1158/1078-0432.CCR-18-1512. Epub 2018 Aug 21.
Puca et al (2019) Int J Cancer. 2020 May 1;146(9):2518-2530.
Car BD et al., Toxicol Pathol. 1999 Jan-Feb;27(1):58-63.

### Sequences

The following sequences form part of the disclosure of the present application. A WIPO ST.25 compatible electronic sequence listing is provided with this application, too. For the avoidance of doubt, if discrepancies exist between the sequences in the following Table 4 and the electronic sequence listing, the sequences in this table shall be deemed to be the correct ones.

| **SEQ ID NO** | **qualifier** | **Sequence** |
|---|---|---|
| 1 | P40 | IWELKKDVYVVELDWYPDAPGEMVVLTCDTPEEDGITWTLDQSSEVLGSGKT LTIQVKEFGDAGQYTCHKGGEVLSHSLLLLHKKEDGIWSTDILKDQKEPKNK TFLRCEAKNYSGRFTCWWLTTISTDLTFSVKSSRGSSDPQGVTCGAATLSAE |
| | | RVRGDNKEYEYSVECQEDSACPAAEESLPIEVMVDAVHKLKYENYTSSFFIR DIIKPDPPKNLQLKPLKNSRQVEVSWEYPDTWSTPHSYFSLTFCVQVQGKSK REKKDRVFTDKTSATVICRKNASISVRAQDRYYSSSWSEWASVPCS |
| 2 | Linker 1 | GGGGSGGGGSGGGGS |
| 3 | P35 | RNLPVATPDPGMFPCLHHSQNLLRAVSNMLQKARQTLEFYPCTSEEIDHEDI TKDKTSTVEACLPLELTKNESCLNSRETSFITNGSCLASRKTSFMMALCLSS IYEDLKMYQVEFKTMNAKLLMDPKRQIFLDQNMLAVIDELMQALNFNSETVP QKSSLEEPDFYKTKIKLCILLHAFRIRAVTIDRVMSYLNAS |
| 4 | Linker 2 ("SAD") | GSADGGSSAGGSDAG |
| 5 | L19VL | |
| 6 | Linker 3/Linker 5 | GSSGG |
| 7 | L19VH | |
| 8 | Linker 4 | SSSSGSSSSGSSSSG |
| 9 | Full length SAD variant | IWELKKDVYVVELDWYPDAPGEMVVLTCDTPEEDGITWTLDQSSEVLGSGKT LTIQVKEFGDAGQYTCHKGGEVLSHSLLLLHKKEDGIWSTDILKDQKEPKNK TFLRCEAKNYSGRFTCWWLTTISTDLTFSVKSSRGSSDPQGVTCGAATLSAE RVRGDNKEYEYSVECQEDSACPAAEESLPIEVMVDAVHKLKYENYTSSFFIR DIIKPDPPKNLQLKPLKNSRQVEVSWEYPDTWSTPHSYFSLTFCVQVQGKSK REKKDRVFTDKTSATVICRKNASISVRAQDRYYSSSWSEWASVPCSGGGGSG GGGSGGGGSRNLPVATPDPGMFPCLHHSQNLLRAVSNMLQKARQTLEFYPCT SEEIDHEDITKDKTSTVEACLPLELTKNESCLNSRETSFITNGSCLASRKTS FMMALCLSSIYEDLKMYQVEFKTMNAKLLMDPKRQIFLDQNMLAVIDELMQA LNFNSETVPQKSSLEEPDFYKTKIKLCILLHAFRIRAVTIDRVMSYLNASGS ADGGSSAGGSDAGEVQLLESGGGLVQPGGSLRLSCAASGFTFSSFSMSWVRQ APGKGLEWVSSISGSSGTTYYADSVKGRFTISRDNSKNTLYLQMNSLRAEDT AVYYCAKPFPYFDYWGQGTLVTVSSGSSGGEIVLTQSPGTLSLSPGERATLS CRASQSVSSSFLAWYQQKPGQAPRLLIYYASSRATGIPDRFSGSGSGTDFTL TISRLEPEDFAVYYCQQTGRIPPTFGQGTKVEIKSSSSGSSSSGSSSSGEVQ LLESGGGLVQPGGSLRLSCAASGFTFSSFSMSWVRQAPGKGLEWVSSISGSS GTTYYADSVKGRFTISRDNSKNTLYLQMNSLRAEDTAVYYCAKPFPYFDYWG QGTLVTVSSGSSGGEIVLTQSPGTLSLSPGERATLSCRASQSVSSSFLAWYQ QKPGQAPRLLIYYASSRATGIPDRFSGSGSGTDFTLTISRLEPEDFAVYYCQ QTGRIPPTFGQGTKVEIK |

**Table 5**

| | **STABILITY STUDY TABLE** | | | |
|---|---|---|---|---|
| **Storage Temperature** | **Time-point** | **Assay (after each thawing and at each time-point)** | **Assay purpose** | **Acceptance criteria** |
| -80°C ± 5°C | stress conditions: | | | • Clear and free of visible particles |
| | 3 cycles of freezing at -80°C and thawing at RT | • Visual appearance | • Detection of macro-precipitation | |
| | | • A₂₈₀ₙₘ | • Detection of protein loss | • Compared to t = 0 value, loss ≤ 10% |
| 2-8°C | t = 7 days of storage | • SEC | • Detection of protein aggregation | |
| | | | | • Compared to t = 0 value, peak of interest loss ≤ 5% |
| | t = 14 days of storage | | | |

**Table 6**

| **Formulation buffer** | **Formulation yield (1) (after desalting)** | **Concentration yield (2) (after concentration)** | **Total yield (1+2)** | **% single chain diabody in the formulated sample (SEC)** | **% single chain diabody in the concentrated sample (SEC)** |
|---|---|---|---|---|---|
| **1A** | 95.0 | 90.7 | 86.2 | 96.08 | 93.15 |
| **1B** | 96.6 | 90.9 | 87.8 | 95.49 | 93.72 |
| **2A** | 98.3 | 88.9 | 87.4 | 95.96 | 94.29 |
| **2B** | 96.6 | 90.4 | 87.3 | 96.11 | 94.18 |
| **3A** | 93.7 | 96.3 | 90.2 | 96.11 | 94.89 |
| **3B** | 96.2 | 93.7 | 90.1 | 95.94 | 94.81 |
| **4A** | 92.6 | 96.7 | 89.5 | 95.95 | 94.72 |

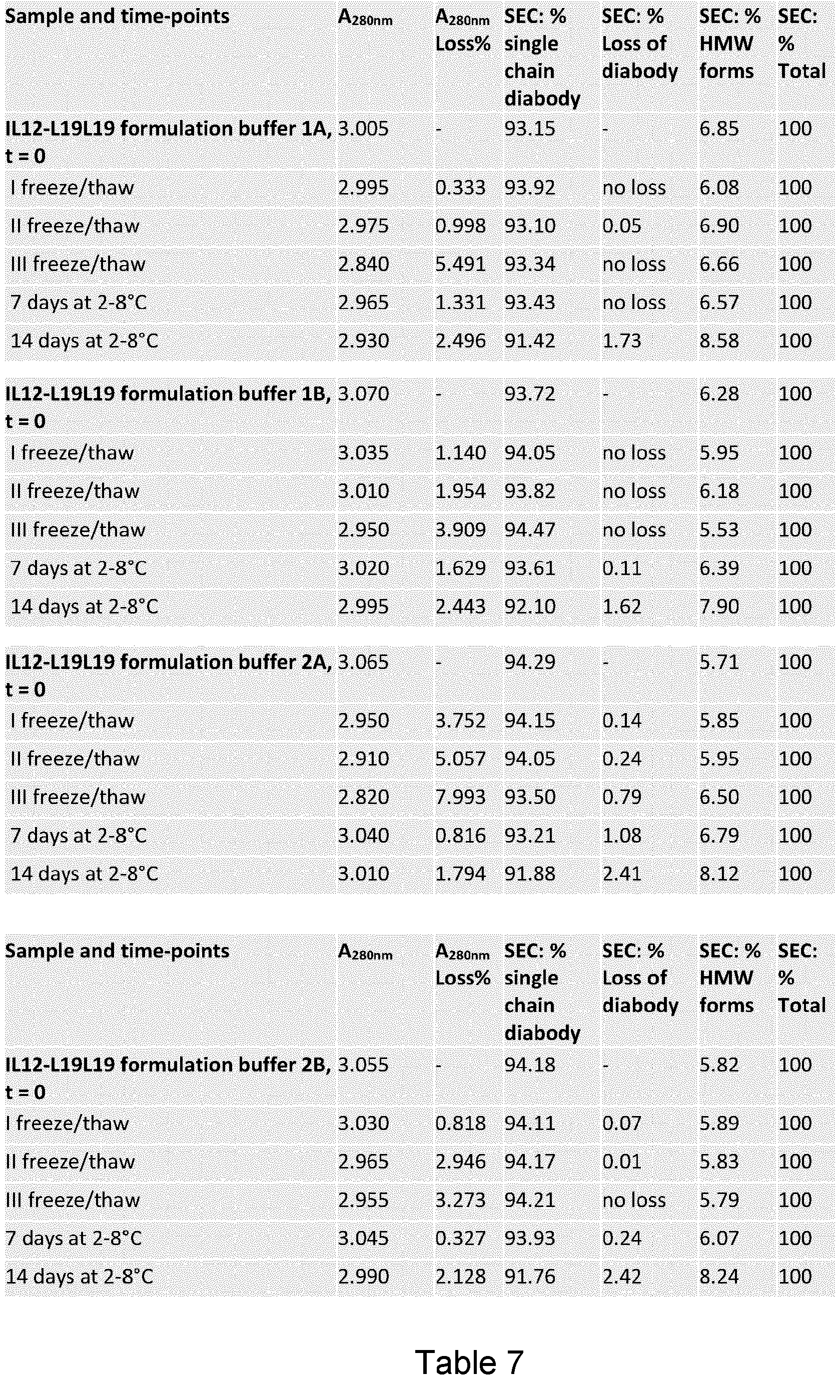

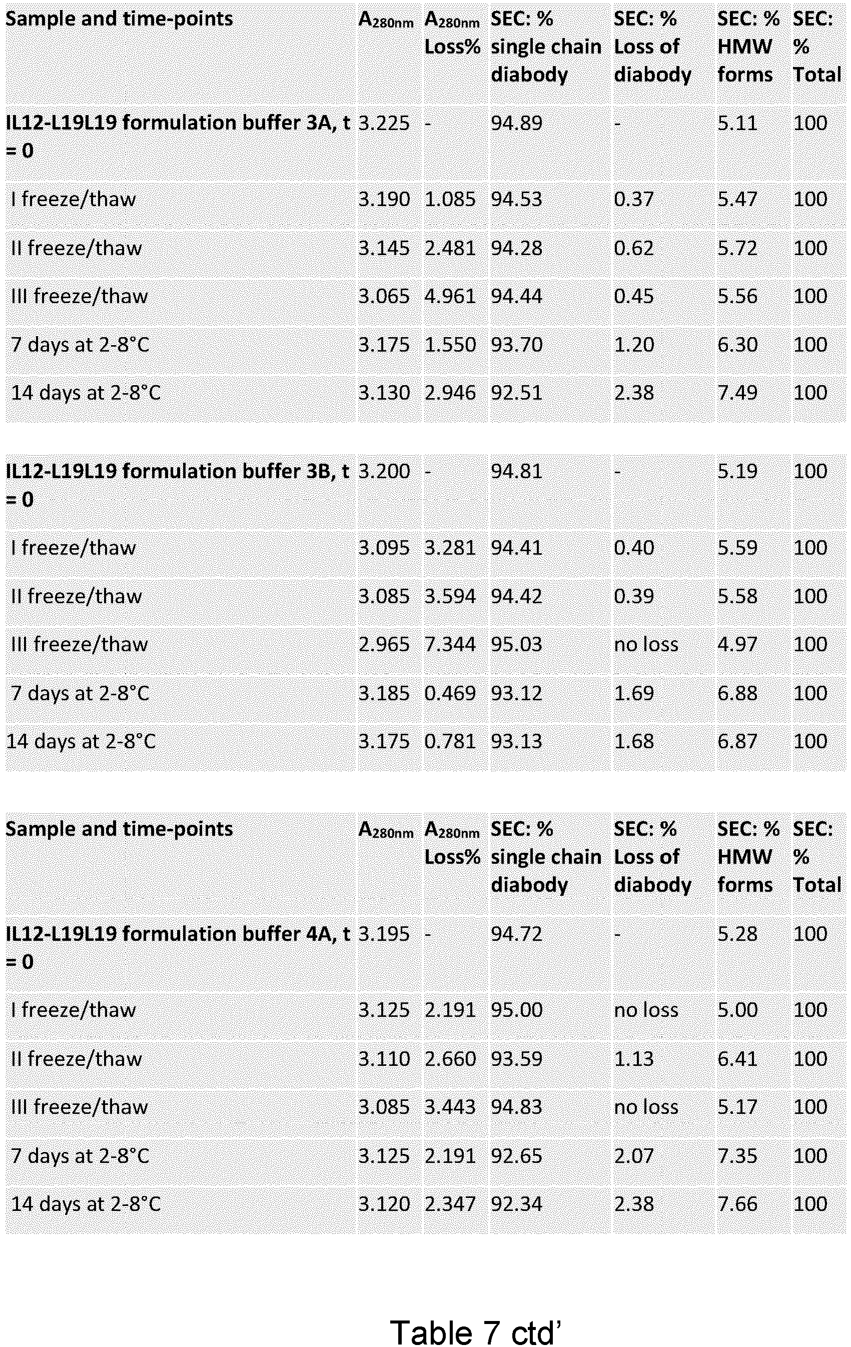

**Table 8**

| **Stability study under stress conditions** | | | | |
|---|---|---|---|---|
| **Classification** | **Formulation buffer** | **% loss of A₂₈₀ₙₘ (3° freeze/thaw cycle)** | **Formulation buffer** | **Excipients in the buffer** |
| 1 | 2B | 3.3% | Histidine | 8.5% w/v Sucrose |
| | | | pH 8.0 | |
| 2 | 4A | 3.4% | Citrate | 8.0% w/v Sucrose |
| | | | pH 6.0 | 1% w/v Glycerol |
| 3 | 1B | 3.9% | Hepes/NaCl pH 7.0 | 50mM Mannitol |
| | | | | 1% w/v Glycerol |
| 4 | 3A | 5.0% | Phosphate pH 6.5 | 50mM Mannitol |
| | | | | 1% w/v Glycerol |
| 5 | 1A | 5.5% | Hepes pH 7.0 | 50mM Mannitol |
| | | | | 1% w/v Glycerol |
| 6 | 3B | 7.3% | Phosphate pH 7.5 | 50mM Mannitol |
| | | | | 1% w/v Glycerol |
| 7 | 2A | 8.0% | Histidine | 8.5% w/v Sucrose |
| | | | pH 6.0 | |

**Table 9**

| **Stability study at 2-8°C** | | | | |
|---|---|---|---|---|
| **Classification** | **Formulation buffer** | **% loss of A₂₈₀ₙₘ (after 14 days)** | **Formulation buffer** | **Excipients in the buffer** |
| 1 | 3B | 0.8% | Phosphate | 50mM Mannitol |
| | | | pH 7.5 | 1% w/v Glycerol |
| 2 | 2A | 1.8% | Histidine | 8.5% w/v Sucrose |
| | | | pH 6.0 | |
| 3 | 2B | 2.1% | Histidine | 8.5% w/v Sucrose |
| | | | pH 8.0 | |
| 4 | 4A | 2.3% | Citrate | 8.0% w/v Sucrose |
| | | | pH 6.0 | 1% w/v Glycerol |
| 5 | 1B | 2.4% | Hepes/NaCl pH 7.0 | 50mM Mannitol |
| | | | | 1% w/v Glycerol |
| 6 | 1A | 2.5% | Hepes | 50mM Mannitol |
| | | | pH 7.0 | 1% w/v Glycerol |
| 7 | 3A | 2.9% | Phosphate pH 6.5 | 50mM Mannitol |
| | | | | 1% w/v Glycerol |

## Claims

1. A pharmaceutical formulation comprising a recombinant protein comprising (i) interleukin-12 (IL-12) and (ii) an antibody binding the extra-domain B (ED-B) of fibronectin, or a target binding fragment thereof, the formulation comprising:
• Histidine, Sucrose and EDTA, adjusted to have a pH of 8.0 ± 0.3
• citric acid, sodium citrate, Sucrose, Glycerol, EDTA, adjusted to have a pH of 6.0 ± 0.3; or
• Hepes, NaCl, Mannitol, Glycerol, EDTA, adjusted to have a pH of 7.0 ± 0.3.

2. The formulation according to claim 1, wherein the antibody comprised in the recombinant protein comprises at least one single-chain Fv (scFv) antibody fragment, optionally a single chain diabody.

3. The formulation according to any one of claims 1 or 2, wherein the IL-12 comprised in the recombinant protein comprises a p40 subunit and a p35 subunit, linked by a linker.

4. The formulation according to any one of claims 1-3, wherein at least one of
• the p40 subunit and the p35 subunit comprise the amino acid sequence according to SEQ ID NO: 1 or SEQ ID NO: 3, respectively,
• the antibody comprised in the recombinant protein is the anti EDB antibody L19, comprising the amino acid sequence according to SEQ ID NO: 5 as VL domain and SEQ ID NO: 7 as VH domain,
• the recombinant protein comprises, optionally consists of, the sequence according to SEQ ID NO: 9.

5. The formulation according to any one of claims 1-4, wherein the recombinant protein comprises
a) a p40 domain linked to a p35 domain by a first linker;
b) a first L19 VH domain linked to the p35 domain by a SAD linker;
c) a first L19 VL domain linked to the first L19 VH domain by a third linker;
d) a second L19 VH domain linked to the first L19 VL domain by a fourth linker; and/or
e) a second L19 VL domain linked to the second L19 VH domain by a fifth linker.

6. The formulation according to any one claims 1-5, which is for intravenous or subcutaneous administration.

## Patentansprüche

1. Eine pharmazeutische Formulierung, umfassend ein rekombinantes Protein umfassend (i) Interleukin 12 (IL-12) und (ii) einen Antikörper, der an die extrazelluläre B-Domäne (ED-B) von Fibronektin bindet, oder ein ziel-bindendes Fragment davon, wobei die Formulierung umfasst:
• Histidin, Saccharose und EDTA, eingestellt auf einen pH-Wert von 8.0 ± 0.3;
• Zitronensäure, Natriumzitrat, Saccharose, Glyzerin, EDTA, eingestellt auf einen pH-Wert von 6.0 ± 0.3; oder
• Hepes, NaCl, Mannitol, Glyzerin, EDTA, eingestellt auf einen pH-Wert von 7.0 ± 0.3.

2. Die Formulierung gemäß Anspruch 1, wobei der Antikörper, der im besagten rekombinanten Protein enthalten ist, mindestens ein Einzel-Ketten Variables Antikörper-Fragment (scFv), vorzugsweise ein Einzel-Ketten-Diabody, umfasst.

3. Die Formulierung gemäß einem der Ansprüche 1 oder 2, wobei das IL-12, das im besagten rekombinanten Protein enthalten ist, eine p40-Untereinheit und eine p35-Untereinheit umfasst, welche durch einen Linker miteinander verbunden sind.

4. Die Formulierung gemäß einem der Ansprüche 1 bis 3, wobei mindestens
• eine der p40- und der p35-Untereinheiten die Aminosäure-Sequenz gemäß SEQ ID Nr. 1 oder SEQ ID Nr. 3 umfasst,
• der Antikörper, der im besagten rekombinanten Protein enthalten ist, der Anti-ED-B-Antikörper L19 ist, umfassend die Aminosäure-Sequenz gemäß SEQ ID Nr. 5 als VL-Domäne und gemäß SEQ ID Nr. 7 als VH-Domäne,
• das rekombinante Protein die Sequenz gemäß SEQ ID Nr. 9 umfasst, bzw. optional aus der Sequenz gemäß SEQ ID Nr. 9 besteht.

5. Die Formulierung gemäß einem der Ansprüche 1 bis 4, wobei das rekombinante Protein umfasst
a) eine p40-Domäne, verbunden mit einer p35-Domäne über einen ersten Linker;
b) eine erste L19 VH-Domäne, verbunden mit einer p35-Domäne über einen SAD-Linker;
c) eine erste L19 VL-Domäne, verbunden mit der ersten L19 VH-Domäne über einen dritten Linker;
d) eine zweite L19 VH-Domäne, verbunden mit der ersten L19 VL-Domäne über einen vierten Linker; und/oder
e) eine zweite L19 VL-Domäne, verbunden mit der zweiten L19 VH-Domäne über einen fünften Linker.

6. Die Formulierung gemäß einem der Ansprüche 1 bis 5, welche für eine intravenöse oder subkutane Verabreichung geeignet ist.

## Revendications

1. Formulation pharmaceutique comprenant une protéine recombinante comprenant (i) l'interleukine-12 (IL-12) et (ii) un anticorps se liant au domaine B supplémentaire (ED-B) de la fibronectine, ou un fragment de liaison cible de ceux-ci, la formulation comprenant :
• histidine, saccharose et EDTA, ajustés à un pH de 8,0 ± 0,3 ;
• acide citrique, citrate de sodium, saccharose, glycérol, EDTA, ajustés à un pH de 6,0 ± 0,3 ; ou
• hepes, NaCl, mannitol, glycérol, EDTA, ajustés à un pH de 7,0 ± 0,3.

2. Formulation selon la revendication 1, dans laquelle l'anticorps compris dans la protéine recombinante comprend au moins un fragment d'anticorps à chaîne unique (scFv), de manière optionnelle un anticorps dimérique à chaîne unique.

3. Formulation selon l'une quelconque des revendications 1 ou 2, dans laquelle l'IL-12 comprise dans la protéine recombinante comprend une sous-unité p40 et une sous-unité p35, liées par un lieur.

4. Formulation selon l'une quelconque des revendications 1 à 3, dans laquelle au moins un de:
• la sous-unité p40 et la sous-unité p35 comprennent respectivement la séquence d'acides aminés selon SEQ ID n° : 1 ou SEQ ID n° : 3,
• l'anticorps compris dans la protéine recombinante est l'anticorps anti-EDB L19, comprenant la séquence d'acides aminés selon SEQ ID n° : 5 en tant que domaine VL et SEQ ID n° : 7 en tant que domaine VH,
• la protéine recombinante comprend, de manière optionnelle consiste en, la séquence selon SEQ ID n° : 9.

5. Formulation selon l'une quelconque des revendications 1 à 4, dans laquelle la protéine recombinante comprend :
a) un domaine p40 lié à un domaine p35 par un premier lieur ;
b) un premier domaine VH L19 lié au domaine p35 par un lieur SAD ;
c) un premier domaine VL L19 lié au premier domaine VH L19 par un troisième lieur ;
d) un second domaine VH L19 lié au premier domaine VL L19 par un quatrième lieur ; et/ou
e) un second domaine VL L19 lié au second domaine VH L19 par un cinquième lieur.

6. Formulation selon l'une quelconque des revendications 1 à 5, destinée à une administration intraveineuse ou sous-cutanée.
